# FASCICULE DE BREVET EUROPEEN

(11) **EP 2 776 482 B1**
(45) Date de publication et mention de la délivrance du brevet: **07.10.2015**
(21) Numéro de dépôt: 12780502.6
(22) Date de dépôt: 05.11.2012
(51) Int. Cl.: C08F 292/00, C07C 321/24, C08F 2/38, H01M 4/92, H01M 4/90

(54) **PROCEDE DE PREPARATION DE PARTICULES APTES A CATALYSER LA REDUCTION DE L'OXYGENE OU L'OXYDATION DE L'HYDROGENE CONDUCTRICES DE PROTONS**
VERFAHREN ZUR HERSTELLUNG PROTONENLEITENDER PARTIKEL ZUR KATALYSIERUNG DER REDUKTION VON SAUERSTOFF ODER ZUR OXYDATION VON WASSERSTOFF
METHOD FOR PREPARING PROTON-CONDUCTING PARTICLES CAPABLE OF CATALYZING THE REDUCTION OF OXYGEN OR THE OXIDATION OF HYDROGEN

(30) Priorité: 07.11.2011 FR 1160115
(43) Date de publication de la demande: 17.09.2014
(73) Titulaire: Commissariat à l'Énergie Atomique et aux Énergies Alternatives, 75015 Paris (FR); Centre National de la Recherche Scientifique, 75016 Paris (FR)
(72) Inventeur: BUVAT, Pierrick, F-37250 Montbazon (FR); FERRANDEZ, Anne-Claire, F-14880 Colleville Montgomery (FR); BARENTON, Steve, F-86000 Poitiers (FR); COUTANCEAU, Christophe, F-86000 Poitiers (FR)
(74) Mandataire: Ahner, Philippe
(86) Numéro de dépôt international: PCT/EP2012/071858
(87) Numéro de publication internationale: WO 2013/068318

(56) Documents cités:
- FRANÇOIS GAL ET AL: "Water-soluble polymer-grafted platinum nanoparticles for the subsequent binding of enzymes. synthesis and SANS", JOURNAL OF POLYMER SCIENCE PART A: POLYMER CHEMISTRY, vol. 50, no. 2, 14 octobre 2011 (2011-10-14), pages 289-296, XP055030754, ISSN: 0887-624X, DOI: 10.1002/pola.25030
- Li Dongxiang: "Fabrication of pH-Responsive Nanocomposites of Gold Nanoparticles/Poly(4-vinylpyridine)", Chem.Mater, 13 janvier 2007 (2007-01-13), pages 412-417, XP055030761, DOI: 10.1021/cm062290+ Extrait de l'Internet: URL:http://pubs.acs.org/doi/pdfplus/10.102 1/cm062290+ [extrait le 2012-06-22]
- GÉRALDINE CARROT ET AL: "Polymer-Grafted-Platinum Nanoparticles: From Three-Dimensional Small-Angle Neutron Scattering Study to Tunable Two-Dimensional Array Formation", LANGMUIR, vol. 25, no. 1, 6 janvier 2009 (2009-01-06), pages 471-478, XP055030798, ISSN: 0743-7463, DOI: 10.1021/la802862q

## Description

### DOMAINE TECHNIQUE

La présente invention se rapporte à un procédé de préparation de particules spécifiques aptes à catalyser la réduction de l'oxygène ou l'oxydation de l'hydrogène, ces particules étant, de plus, conductrices de protons grâce à une fonctionnalisation desdites particules avec des polymères organiques conducteurs de protons.

La présente invention se rapporte également aux particules obtenues par ce procédé.

Ces particules ont pour caractéristique de présenter une activité catalytique (notamment, pour l'oxydation de l'hydrogène ou la réduction de l'oxygène) tout en présentant une conductivité protonique.

De ce fait, ces particules trouvent leur application dans l'élaboration de matériaux d'électrodes, en particulier de matériaux destinés à entrer dans la constitution de couches catalytiques d'électrodes pour piles à combustible, telles que les piles fonctionnant à H₂/air ou à H₂/O₂ (connues sous l'abréviation PEMFC signifiant « Proton Exchange Membrane Fuel Cell »).

Ainsi, la présente invention se situe dans le domaine des piles à combustible fonctionnant sur le principe de l'oxydation de l'hydrogène et la réduction de l'oxygène.

### ETAT DE LA TECHNIQUE ANTÉRIEURE

Une pile à combustible de ce type est un générateur électrochimique, qui convertit l'énergie chimique en énergie électrique grâce à deux réactions électrochimiques : une réaction d'oxydation à l'anode d'un combustible (l'hydrogène) combinée à une réaction de réduction à la cathode d'un comburant (l'air ou l'oxygène).

Classiquement, ce type de piles à combustible comporte une pluralité de cellules électrochimiques montées en série, chaque cellule comprenant deux électrodes de polarité opposée séparées par une membrane échangeuse de protons faisant office d'électrolyte solide, cette membrane assurant le passage vers la cathode des protons formés, par réaction électrochimique, lors de l'oxydation du combustible à l'anode.

Les réactions électrochimiques susmentionnées (oxydation et réduction) se produisent au niveau de zones spécifiques des électrodes (dites, zones actives correspondant structurellement à des couches catalytiques) qui forment la jonction entre la couche de diffusion (au niveau de laquelle se produit l'alimentation en réactifs) des électrodes et la membrane et nécessitent, pour se produire, l'utilisation de catalyseurs, qui consistent, classiquement, pour les piles du type PEMFC, en des particules de platine.

Compte tenu des coûts impliqués par la présence d'un catalyseur comme le platine, il convient d'obtenir un maximum de surface catalytique pour une masse donnée de métal, un tel objectif pouvant être atteint par des particules de platine de tailles nanométriques (dénommées également nanoparticules de platine).

Il convient également, pour que les réactions électrochimiques puissent avoir lieu, que les particules de platine soient en contact à la fois avec du combustible ou du comburant (selon que l'on se situe au niveau de l'anode ou de la cathode), du conducteur protonique constitutif de la membrane et du conducteur électronique entrant dans la constitution de l'électrode (ce conducteur électronique étant classiquement un matériau carboné), cette zone de contact étant connue sous l'appellation de point triple, l'électrode étant d'autant plus efficace que le nombre de points triples est élevé.

En d'autres termes, en ces points triples, se présentent au niveau des particules de platine :
- une continuité physique avec la membrane électrolytique, pour assurer une conduction des protons H⁺ ;
- une continuité physique avec le conducteur électronique, pour assurer la conduction des électrons ; et
- une continuité physique avec la zone de diffusion des électrodes, pour assurer la diffusion des gaz (l'oxygène ou l'hydrogène pour les piles PEMFC).

Le maintien dans le temps de ces points triples suppose le respect de l'intégrité des zones de contacts entre les différents éléments entrant dans la constitution de ces points triples, ce qui implique un maintien de l'intégrité physique de ces différents éléments, notamment des particules de platine.

Or certaines études ont montré qu'il est possible d'assister, en cours de fonctionnement d'une pile, à une dégradation des particules de platine (induisant, de ce fait, une diminution de surface active) soit par des phénomènes de dissolution ou des phénomènes d'augmentation de tailles de particules (découlant, classiquement, de phénomènes d'agglomération).

Ces phénomènes de dissolution peuvent se produire avec des piles fonctionnant à très faibles pH (par exemple, un pH inférieur à 1) et à potentiels élevés de fonctionnement à la cathode (par exemple, un potentiel supérieur à 1 V par rapport à ERH (ERH signifiant électrode réversible à dihydrogène) le platine dissous pouvant se retrouver soit dans l'eau formée au cours du fonctionnement de la pile soit à l'intérieur de la membrane électrolytique, généralement, polymérique, ce qui conduit, en son sein, à la formation de nanocristaux de platine inactifs.

Quant aux phénomènes d'augmentation, ils peuvent se produire avec des piles dont les nanoparticules de platine présentent une mobilité importante à la surface du support généralement carboné, sur lequel elles sont déposées, cette mobilité dépendant de l'énergie de surface de celui-ci.

Pour contourner ces phénomènes, il peut être fait appel à de forts taux de chargement en particules de platine avec les inconvénients que cela représente en termes de coûts de production, eu égard au prix très élevé du platine sur les marchés.

Afin de diminuer les taux de chargement tout en accédant à une surface active efficace, les études ont porté sur l'optimisation des assemblages électrode (ici, comprenant des particules de platine)-membrane.

Ainsi, il a été proposé de juxtaposer, par contact intime, les différents éléments (particules de platine, conducteur électrique et électrolyte) nécessaires à la création des points triples, cette juxtaposition pouvant consister :
- à mélanger des particules de platine avec de la poudre de carbone (remplissant le rôle de conducteur électrique) et à imprégner l'ensemble avec de l'électrolyte, de sorte à garantir un meilleur contact avec la membrane ;
- à déposer par des techniques de dépôts de couches minces (telles que l'électrodéposition ou la pulvérisation par voie physique) des particules de platine, ce qui permet de déposer du platine selon de faibles concentrations tout en conservant une activité catalytique très élevée.

Toutefois, les ensembles résultant de ces techniques sont fragiles en raison des liaisons faibles impliquées pour juxtaposer les éléments constitutifs de ces ensembles, ce qui ne permet pas d'empêcher les phénomènes de dégradation dus à la migration des particules de platine occasionnant, de ce fait, une diminution de la durée de vie de ces ensembles.

Au vu de ce qui précède, les auteurs de la présente invention se sont fixé pour objectif de proposer un procédé de fabrication de particules comprenant un matériau apte à catalyser l'oxydation de l'hydrogène ou la réduction de l'hydrogène, lesquelles particules sont liées à un conducteur protonique (en l'occurrence des polymères conducteurs de protons) et, éventuellement à un conducteur électronique (tel qu'un matériau carboné) par des liaisons plus fortes que les ensembles existant dans l'art antérieur, de sorte à améliorer la pérennité des points triples, lorsque ces particules sont destinées à être utilisées pour la constitution de couches catalytiques de piles à combustible du type PEMFC.

### EXPOSÉ DE L'INVENTION

Ainsi, l'invention a trait à un procédé de préparation de particules comprenant un matériau apte à catalyser la réduction de l'oxygène ou l'oxydation de l'hydrogène, lesdites particules étant fonctionnalisées par des polymères comprenant au moins un motif répétitif porteur d'au moins un groupe conducteur de protons et lesdites particules étant liées, par exemple, par covalence à un matériau carboné, ledit procédé comprenant successivement les étapes suivantes :
a) une étape de mise en contact de particules comprenant un matériau apte à catalyser la réduction de l'oxygène ou l'oxydation de l'hydrogène avec un composé amorceur d'une polymérisation du type ATRP, lequel composé comprend au moins un groupe apte à se greffer à la surface desdites particules, moyennant quoi l'on obtient des particules greffées par un reste dudit composé amorceur ;
b) une étape de mise en contact desdites particules obtenues en a) avec au moins un monomère porteur d'au moins un groupe conducteur de protons, moyennant quoi il y a polymérisation dudit monomère à partir des restes susmentionnés.

Avant d'entrer plus en détail dans la présente description, nous précisons les définitions suivantes.

Par polymère, on entend, classiquement, au sens de l'invention, un composé constitué par l'enchaînement d'un ou plusieurs motifs répétitifs.

Par motif répétitif, on entend, classiquement, au sens de l'invention, un groupe organique bivalent (c'est-à-dire un groupe formant pont) issu d'un monomère après polymérisation de celui-ci.

Par polymérisation du type ATRP, on entend, une polymérisation radicalaire par transfert d'atomes (ATRP correspondant à l'abréviation de la terminologie anglaise « Atom Transfer Radical Polymerization »). Le mécanisme de ce type de polymérisation sera défini plus en détail ci-dessous.

Par composé amorceur d'une polymérisation du type ATRP, on entend un composé comprenant au moins un groupe apte à amorcer ce type de polymérisation, le composé amorceur de polymérisation conforme à l'invention comprenant, en outre, un groupe apte à se greffer à la surface des particules susmentionnées, ce qui signifie en d'autres termes, que ce groupe réagit en présence des particules pour se fixer par covalence à la surface de celles-ci (on peut parler, aussi, de greffage), moyennant quoi l'amorceur subsiste à la surface des particules sous forme d'un reste, étant entendu que ce reste comporte encore au moins un groupe apte à amorcer une polymérisation du type ATRP.

Ainsi, grâce à la mise en oeuvre du procédé de l'invention, il est ainsi possible d'obtenir des particules comprenant un matériau apte à catalyser la réduction de l'oxygène ou l'oxydation de l'hydrogène greffées par covalence via un reste de composé amorceur à des polymères conducteurs de protons, ce qui permet, lorsque ces particules sont destinées à entrer dans la constitution d'électrodes (notamment au niveau de couches catalytiques de celles-ci), d'assurer une bonne continuité physique avec l'électrolyte adjacent, lorsque celui-ci est à base également de polymère(s) conducteur(s) de protons.

Comme mentionné ci-dessous, le procédé de l'invention comporte une étape de mise en contact de particules comprenant un matériau apte à catalyser la réduction de l'oxygène ou l'oxydation de l'hydrogène avec un composé amorceur d'une polymérisation du type ATRP comprenant un groupe apte à se greffer à la surface desdites particules, moyennant quoi l'on obtient des particules greffées par un reste dudit composé (étape a).

Cette étape de mise en contact peut comprendre une opération de dispersion des particules susmentionnées suivie d'une opération de mise en contact de la dispersion obtenue avec un composé amorceur tel que défini ci-dessus.

Les particules comprenant un matériau apte à catalyser la réduction de l'oxygène ou l'oxydation de l'hydrogène peuvent être des particules métalliques, à savoir des particules comprenant un ou plusieurs éléments métalliques (auquel cas, lorsqu'il y a plusieurs éléments métalliques, on pourra parler de particules en alliage(s) métallique(s)).

Des particules métalliques particulièrement appropriées peuvent être des particules comprenant un métal noble, tel que le platine, le ruthénium, le palladium et des mélanges de ceux-ci.

Lorsque les particules obtenues selon l'invention sont destinées à être utilisées dans des piles PEMFC, les particules métalliques sont avantageusement des particules en platine.

Le composé amorceur d'une polymérisation du type ATRP est un composé comprenant au moins un groupe apte à amorcer la polymérisation ATRP, c'est-à-dire un groupe apte à se cliver au niveau d'une liaison pour former une première espèce radicalaire et une deuxième espèce radicalaire, la première espèce radicalaire réagissant ultérieurement, avec un premier carbone porteur d'une double liaison appartenant au monomère mis en oeuvre dans l'étape b), la deuxième espèce radicalaire se fixant à un deuxième atome opposé au premier carbone porteur de la double liaison.

En d'autres termes, ce mécanisme peut être résumé selon le schéma réactionnel suivant :

Z-Y + C=C → Z-C-C-Y

Z-Y correspondant à l'amorceur susmentionné avec Z correspondant à la première espèce et Y correspondant à la deuxième espèce.

Pour des raisons de simplicité, nous avons représenté ci-dessus uniquement la double liaison du monomère impliqué dans l'étape b).

En outre, le composé amorceur utilisé dans le cadre du procédé de l'invention comprend au moins un groupe apte à se greffer à la surface des particules susmentionnées, c'est-à-dire un groupe apte à réagir avec la surface desdites particules pour former une liaison covalente, moyennant quoi il subsiste un reste de cet amorceur lié de façon covalente à la surface desdites particules.

Le composé amorceur utilisé dans le cadre du procédé de l'invention peut être un composé organique halogénure (à savoir, un composé comprenant au moins un atome d'halogène lié à un atome de carbone, le groupe résultant pouvant être symbolisé par -C-X, X représentant un atome d'halogène) comportant au moins un groupe choisi parmi -S-S- et -SH, -S-S- étant un groupe disulfure divalent, c'est-à-dire un groupe disulfure formant pont entre deux autres groupes du composé et -SH étant un groupe monovalent thiol.

Dans ce type de composés, le groupe apte à amorcer une polymérisation du type ATRP est le groupe -C-X mentionné ci-dessus, ce groupe pouvant se cliver, de façon homolytique, au niveau de la liaison carbone-halogène pour former deux espèces radicalaires, une première espèce radicalaire carbone (pouvant être symbolisé par -C˙) et une deuxième espèce radicalaire consistant en un radical halogène (pouvant être symbolisé par X˙), la première espèce réagissant avec une extrémité de la double liaison du monomère et la deuxième espèce réagissant avec l'extrémité opposée de la double liaison.

Le groupe apte à se greffer à la surface de particules consiste, pour ce type de composés, en un groupe choisi parmi -S-S- et -SH.

Lorsqu'il s'agit d'un groupe disulfure -S-S-, le composé amorceur, en présence de particules, va se scinder en deux restes organiques par clivage homolytique de la liaison entre les deux atomes de soufre, les deux restes consistant en des espèces radicalaires, les électrons libres de ces espèces étant situés au niveau des atomes de soufre, ces électrons libres s'associant chacun avec un électron présent à la surface des particules pour former une liaison covalente entre les restes susmentionnés et les particules *via* les atomes de soufre.

Lorsqu'il s'agit d'un groupe thiol -SH, le composé amorceur, en présence de particules, réagit *via* ce groupe avec la surface des particules pour former une liaison covalente avec celles-ci, moyennant quoi il subsiste de l'amorceur un reste organique greffé à la surface desdites particules, ce reste étant de formule identique à l'amorceur mis à part le fait que l'atome d'hydrogène lié à l'atome de soufre est remplacé par une liaison covalente entre le soufre et une particule.

Ce type de composés est particulièrement adapté en vue d'être greffé par covalence à la surface de particules de platine.

Des composés répondant à cette spécificité peuvent être des composés comportant un groupe disulfure -S-S-, en particulier, des composés symétriques, c'est-à-dire des composés comportant une symétrie autour de la liaison disulfure, ce qui signifie, en d'autres termes, que les deux parties du composé situées de part et d'autre de la liaison disulfure sont identiques.

Plus précisément, des composés répondant à cette spécificité peuvent être des composés comportant un groupe disulfure -S-S- formant pont entre deux parties de ces composés, au moins une de ces deux parties comportant un groupe phényle porteur d'un groupe amide -NH-CO-R¹, R¹ étant un groupe hydrocarboné porteur d'au moins un atome d'halogène (il s'entend, dans ce cas, que le groupe amide est lié à un atome de carbone du groupe phényle *via* l'atome d'azote du groupe -NH-).

Les deux parties susmentionnées peuvent être identiques.

Un composé particulier répondant à la définition donnée ci-dessus peut être un composé de formule (I) ci-dessous :

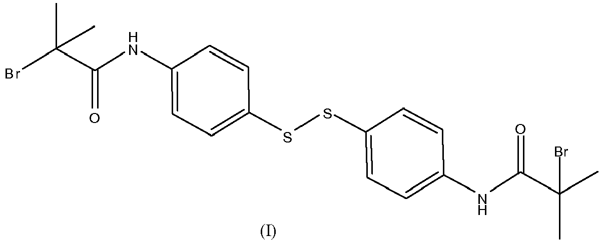

La structure symétrique de ce composé permet de greffer à la surface des particules un radical mercaptoaniline bromé présent de part et d'autre du groupe disulfure, ce qui présente l'avantage d'assurer un greffage total du composé amorceur à la surface des particules.

Ce type de composés peut être synthétisé par une réaction d'acylation entre un composé aminophényledisulfure et un composé chlorure d'acyle, en milieu basique et solvant organique et en présence éventuelle d'un catalyseur, cette réaction d'acylation se produisant selon un mécanisme d'addition-fragmentation.

A titre d'exemple, lorsqu'il s'agit de préparer un composé de formule (I) susmentionnée, la réaction d'acylation peut se produire entre un composé 4-aminophényle disulfure et un composé bromoisobutyrate selon le schéma réactionnel suivant : 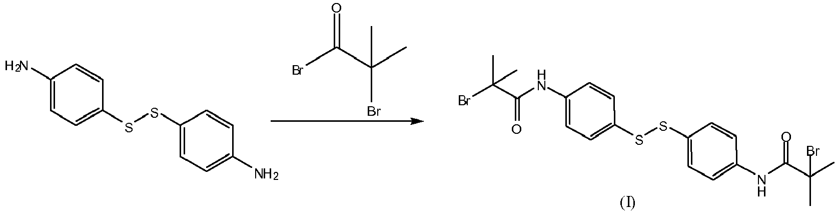 cette réaction pouvant être réalisée avec de la bipyridine, comme catalyseur, du chloroforme comme solvant organique, dans une gamme de températures allant de 0°C à la température ambiante.

La quantité de composé amorceur mise en jeu lors de l'étape a) peut aller de 0,5 à 20%, idéalement de 2 à 10% en masse par rapport par la masse du composé amorceur, des particules et éventuellement du matériau carboné.

Le procédé de l'invention peut comprendre, avant la mise en oeuvre de l'étape a), une étape de préparation desdites particules susmentionnées.

Lorsque les particules sont des particules métalliques, la préparation de ces dernières peut consister à réduire un sel métallique en faisant réagir celui-ci avec un agent réducteur.

Par exemple, lorsque les particules métalliques sont des particules de platine, elles peuvent être préparées par réduction d'un sel de platine avec un agent réducteur.

Le sel de platine peut être un sel d'halogénure de platine, éventuellement hydraté, tel que H₂PtCl₆.6H₂O.

L'agent réducteur peut être un hydrure métallique, et plus particulièrement un borohydrure métallique, tel que le borohydrure de sodium (NaBH₄).

La préparation peut être réalisée dans un milieu du type émulsion « huile-dans-eau » (correspondant à la terminologie anglaise « water-in-oil »), l'huile pouvant correspondre à un composé hydrocarbure, tel que l'hexane.

D'un point de vue pratique, la préparation de particules de platine dans un tel milieu peut se dérouler par la mise en oeuvre des opérations suivantes :
- une opération de mise en contact d'un sel de platine (par exemple, H₂PtCl₆-H₂0) préalablement dissous dans de l'eau avec un milieu comprenant une huile et éventuellement un agent dispersant (par exemple, du tétraéthylèglycoldodécyléther) ;
- une opération d'ajout au mélange résultant de l'opération précédente d'un agent réducteur, en une ou plusieurs fois, à l'issue de laquelle le mélange résultant est agité pendant une durée suffisante jusqu'à cessation de tout dégagement gazeux (cette cessation indiquant que la réaction de réduction est achevée).

Le mélange obtenu comprend ainsi des particules de platine, lequel mélange peut être utilisé tel quel pour la mise en oeuvre de l'étape a) (on pourra ainsi dire que l'étape a) est réalisée *in situ*).

En variante, le mélange obtenu peut être traité (par exemple, par filtration) de sorte à isoler les particules de platine obtenues, ces dernières étant destinées à être utilisées pour la mise en oeuvre de l'étape a).

Outre le fait que les particules obtenues selon le procédé de l'invention sont fonctionnalisées par des polymères comprenant au moins un motif répétitif porteur d'au moins un groupe conducteur de protons, ces particules sont également liées, par exemple, par covalence, à un matériau carboné (pouvant être assimilé à un support carboné), tel que du graphite, du noir de carbone, des fibres de carbone, des tubes de carbone (tels que des nanotubes de carbone), du graphène et des mélanges de ceux-ci.

La liaison des particules à un matériau carboné peut intervenir, selon des variantes, à différents moments de la mise en oeuvre du procédé de l'invention.

Selon un premier mode de réalisation, les particules peuvent être utilisées déjà liées à un matériau carboné lors de la mise en oeuvre de l'étape a).

Ces particules déjà liées à un matériau carboné peuvent être préparées préalablement à l'étape de mise en oeuvre de l'étape a).

Dans ce cas, le procédé de l'invention peut comprendre, avant la mise en oeuvre de l'étape a), une étape de préparation de particules comprenant un matériau apte à catalyser la réduction de l'oxygène ou l'oxydation de l'hydrogène liées à un matériau carboné.

Selon une première variante, lorsque les particules sont des particules métalliques, la préparation de ces dernières peut comprendre :
- une opération de réduction d'un sel métallique en faisant réagir celui-ci avec un agent réducteur, moyennant quoi l'on obtient des particules métalliques ;
- une opération de mise en contact du milieu résultant de l'opération précédente avec le matériau carboné destiné à être lié aux particules, moyennant quoi l'on obtient des particules métalliques liées à un matériau carboné.

Par exemple, lorsque les particules métalliques sont des particules de platine, l'opération de réduction consiste à réduire un sel de platine avec un agent réducteur.

Le sel de platine peut être un sel d'halogénure de platine, éventuellement hydraté, tel que H₂PtCl₆.6H₂O.

L'agent réducteur peut être un hydrure métallique, et plus particulièrement un borohydrure métallique, tel que le borohydrure de sodium (NaBH₄).

La préparation peut être effectuée dans un milieu du type émulsion « huile-dans-eau » (correspondant à la terminologie anglaise « water-in-oil »), l'huile pouvant correspondre à un composé hydrocarbure, tel que l'hexane.

D'un point de vue pratique, la préparation de particules de platine dans un tel milieu, avant la mise en contact avec le matériau carboné, peut se dérouler par la mise en oeuvre des opérations suivantes :
- une opération de mise en contact d'un sel de platine (par exemple, H₂PtCl₆-H₂O) préalablement dissous dans de l'eau avec un milieu comprenant une huile et éventuellement un agent dispersant;
- une opération d'ajout au mélange résultant de l'opération précédente d'un agent réducteur, à l'issue de laquelle le mélange résultant est agité pendant une durée suffisante jusqu'à cessation de tout dégagement gazeux (cette cessation indiquant que la réaction de réduction est achevée), moyennant quoi l'on obtient un mélange comprenant des particules de platine.

Ce mélange comprenant des particules de platine est mis ensuite en contact avec le matériau carboné, de préférence sous ultrasons, moyennant quoi l'on obtient, à l'issue de cette opération de mise en contact, un mélange final comprenant des particules de platine liées (par exemple, par covalence) au matériau carboné, lequel mélange peut être utilisé tel quel pour la mise en oeuvre de l'étape a) (on pourra ainsi dire que l'étape a) est réalisée *in situ*).

En variante, ce mélange comprenant des particules de platine peut être traité (par exemple, par filtration) de sorte à isoler les particules de platine obtenues, ces dernières étant destinées à être utilisées pour la mise en oeuvre de l'étape a).

Selon une deuxième variante, lorsque les particules sont des particules métalliques, la préparation peut consister en une étape de réduction au moyen d'un agent réducteur d'un mélange comprenant un sel métallique et un matériau carboné, en faisant réagir celui-ci avec un agent réducteur, moyennant quoi l'on obtient des particules métalliques liées au matériau carboné.

Par exemple, lorsque les particules métalliques sont des particules de platine, l'étape de réduction consiste à réduire un sel de platine avec un agent réducteur.

Le sel de platine peut être un sel d'halogénure de platine, éventuellement hydraté, tel que H₂PtCl₆.6H₂O.

L'agent réducteur peut être un hydrure métallique, et plus particulièrement un borohydrure métallique, tel que le borohydrure de sodium (NaBH₄).

Encore plus spécifiquement, la préparation de particules de platine liées à un matériau carboné peut se dérouler par la mise en oeuvre des opérations suivantes :
- une opération de mise en contact d'une solution aqueuse basique (par exemple, une solution aqueuse à base de carbonate de lithium) d'un sel de platine (par exemple, H₂PtCl₆-H₂0) avec le matériau carboné ;
- une opération d'ajout au mélange résultant de l'opération précédente d'un agent réducteur, à l'issue de laquelle le mélange résultant est agité pendant une durée suffisante jusqu'à obtention d'un mélange comprenant des particules de platine liées au matériau carboné.

Ce mélange comprenant des particules de platine peut être utilisé tel quel pour la mise en oeuvre de l'étape a) ou peut être traité (par exemple, par filtration) de sorte à isoler les particules de platine obtenues, ces dernières étant destinées à être utilisées pour la mise en oeuvre de l'étape a).

Selon un deuxième mode de réalisation, les particules peuvent être utilisées, lors de la mise en oeuvre de l'étape a), sous une forme non liée au matériau carboné, ce qui implique, dans ce cas, que le procédé de l'invention comprenne, après l'étape a) et avant l'étape b), une étape de mise en contact des particules obtenues à l'issue de l'étape a) avec le matériau carboné (dite ci-après étape a'), moyennant quoi lesdites particules sont liées à l'issue de cette étape au matériau carboné.

Plus spécifiquement, cette étape de mise en contact peut être réalisée sous ultrasons, de sorte à activer la collision des particules avec le matériau carboné pour former une liaison entre ces particules et le matériau.

Pour le premier mode de réalisation (c'est-à-dire le mode de réalisation, dans lequel les particules sont utilisées déjà liées à un matériau carboné avant la mise en oeuvre de l'étape a)), l'étape a) peut être réalisée par les opérations suivantes :
- une opération de mise en dispersion des particules dans un solvant organique électrophile, tel qu'un solvant amine (comme l'hexylamine), ce type de solvant contribuant à assurer une bonne stabilisation de la dispersion, moyennant quoi l'on obtient une dispersion de particules comprenant un matériau apte à catalyser l'oxydation de l'hydrogène ou la réduction de l'oxygène liées à un matériau carboné ;
- une opération de mise en contact de la dispersion susmentionnée avec un composé amorceur tel que défini ci-dessus, de préférence préalablement dissous dans un solvant ou mélange de solvants compatible avec le solvant utilisé pour la dispersion (par exemple, un mélange solvant amine/alcool, tel qu'un mélange hexylamine/méthanol (50/50), lorsque le solvant de dispersion utilisé est l'hexylamine), de sorte à ne pas perturber la dispersion lors de l'opération de mise en contact ;
- éventuellement, une opération de lavage des particules obtenues, par exemple, par des cycles de précipitation/centrifugation, de sorte à éliminer les traces de composé amorceur n'ayant pas réagi.

Pour le deuxième mode de réalisation (c'est-à-dire le mode de réalisation, pour lequel le procédé de l'invention comprend, après l'étape a) et avant l'étape b), une étape de mise en contact des particules obtenues à l'issue de l'étape a) avec le matériau carboné (dite ci-après étape a'), moyennant quoi lesdites particules sont liées à l'issue de cette étape au matériau carboné), le procédé de l'invention peut comprendre :
- une étape de préparation de particules comprenant un matériau apte à catalyser l'oxydation de l'hydrogène ou la réduction de l'oxygène ;
- une étape a), telle que définie ci-dessus, de mise en contact, dans le milieu de synthèse de l'étape précédente, d'un composé amorceur d'une polymérisation ATRP avec les particules obtenues préalablement ;
- une étape de mise en contact des particules issues de l'étape a) avec un matériau carboné, de sorte à obtenir des particules liées à un matériau carboné et greffées par des restes de composé amorceur.

Lorsque les particules sont des particules métalliques, l'étape de préparation de ces dernières peut comprendre une opération de réduction d'un sel métallique en faisant réagir celui-ci avec un agent réducteur, moyennant quoi l'on obtient des particules métalliques.

Par exemple, lorsque les particules métalliques sont des particules de platine, l'étape de réduction consiste à réduire un sel de platine avec un agent réducteur.

Le sel de platine peut être un sel d'halogénure de platine, éventuellement hydraté, tel que H₂PtCl₆.6H₂O.

L'agent réducteur peut être un hydrure métallique, et plus particulièrement un borohydrure métallique, tel que le borohydrure de sodium (NaBH₄).

La préparation peut être effectuée dans un milieu du type émulsion « huile-dans-eau » (correspondant à la terminologie anglaise « water-in-oil »), l'huile pouvant correspondre à un composé hydrocarbure, tel que l'hexane.

D'un point de vue pratique, la préparation de particules de platine dans un tel milieu, avant la mise en contact avec le composé amorceur puis le matériau carboné, peut se dérouler par la mise en oeuvre des opérations suivantes :
- une opération de mise en contact d'un sel de platine (par exemple, H₂PtCl₆-H₂0) préalablement dissous dans de l'eau avec un milieu comprenant une huile et éventuellement un agent dispersant;
- une opération d'ajout au mélange résultant de l'opération précédente d'un agent réducteur, à l'issue de laquelle le mélange résultant est agité pendant une durée suffisante jusqu'à cessation de tout dégagement gazeux (cette cessation indiquant que la réaction de réduction est achevée), moyennant quoi l'on obtient un mélange comprenant des particules de platine.

L'étape a) de mise en contact peut se faire par introduction du composé amorceur en milieu aqueux, de sorte à ne pas perturber le milieu de synthèse des particules.

L'étape de mise en contact avec le matériau carboné peut se faire par introduction de celui-ci directement dans le milieu de synthèse et soumission du mélange résultant à un traitement aux ultrasons, de sorte à engendrer la liaison du matériau carboné aux particules.

Ce mode de réalisation permet de réaliser une synthèse monotope de particules liées à un matériau carboné et greffées à des restes de composé amorceur d'une polymérisation ATRP.

Une fois l'étape a) mise en oeuvre et l'éventuelle étape a'), le procédé de l'invention comprend une étape de mise en contact desdites particules obtenues en a) avec un monomère porteur d'au moins un groupe conducteur de protons, moyennant quoi il y a polymérisation dudit monomère à partir des restes susmentionnés, à l'issue de laquelle il résulte des particules fonctionnalisées par des polymères comprenant au moins un motif répétitif porteur d'au moins un groupe conducteur de protons, *via* des restes de composé amorceur.

Le groupe conducteur de protons peut être un groupe acide sulfonique -SO₃H, un groupe acide carboxylique -CO₂H ou un groupe acide phosphonique -PO₃H₂, ces groupes pouvant être présents éventuellement sous forme de sels.

Les monomères aptes à être utilisés dans le cadre de l'étape b) peuvent être tous types de monomères compatibles avec une polymérisation du type ATRP et comprenant au moins un groupe conducteur de protons éventuellement sous forme d'un sel.

Ces monomères peuvent être notamment choisis de sorte, après polymérisation, à former des chaînes polymériques appartenant à la famille des polysulfones, polyéthercétones, polyphénylènes, polystyrènes, polymères aliphatiques fluorés, étant entendu que ces polymères doivent comporter des groupes conducteurs de protons, par exemple des groupes acide sulfonique, acide phosphonique ou acide carboxylique.

En particulier, des monomères peuvent être :
*des monomères éthyléniques porteurs d'au moins un groupe acide sulfonique, éventuellement sous forme d'un sel ;
*des monomères éthyléniques porteurs d'au moins un groupe acide carboxylique, éventuellement sous forme d'un sel ; et
*des monomères éthyléniques porteurs d'au moins un groupe acide phosphonique, éventuellement sous forme d'un sel.

Ces monomères peuvent être éventuellement fluorés.

Encore plus particulièrement, ces monomères peuvent répondre à la formule (II) suivante : 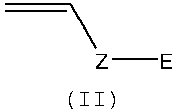 dans laquelle :
- Z correspond à un groupe phénylène ; et
- E correspond à un groupe conducteur de protons, éventuellement sous forme d'un sel, tel qu'un groupe acide sulfonique, un groupe acide phosphonique ou un groupe acide carboxylique.

Un monomère spécifique répondant à la définition donnée ci-dessus est un monomère acidestyrènesulfonique, par exemple sous forme d'un sel, tel qu'un sel de sodium (auquel cas, on pourra parler de styrènesulfonate de sodium).

Un exemple de ce type de monomère est un monomère de formule (III) suivante : 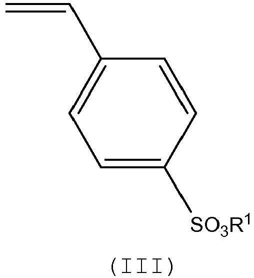 dans laquelle R¹ est un atome d'hydrogène ou un cation (par exemple, un cation de métal alcalin).

Comme mentionné ci-dessus, l'étape de polymérisation b) est régie par les mécanismes de la polymérisation ATRP, qui fonctionne sur le principe de la formation réversible et rapide d'espèces dites « espèces dormantes » par création d'une liaison covalente avec l'espèce radicalaire réactive.

Le schéma réactionnel de ce type de polymérisation peut être illustré par le schéma réactionnel suivant : 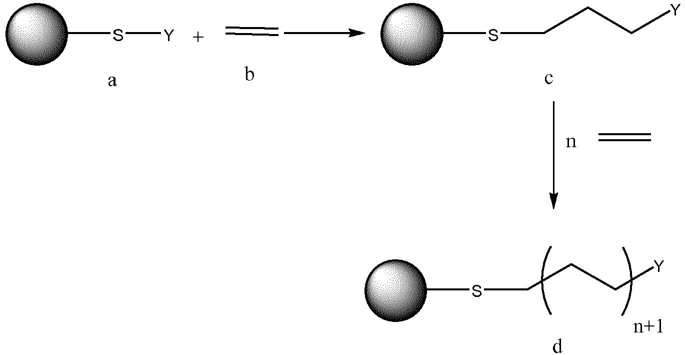 le groupement -S-Y illustrant schématiquement le reste de composé amorceur lié à la surface d'une particule (représentée ici par une sphère pleine), le composé = (indiqué par b) illustrant un monomère.

Comme il ressort de ce schéma, la chaîne polymérique croit par additions successives de monomères sur des radicaux libres, comme dans une polymérisation radicalaire classique, les radicaux libres étant créés par départ du groupe Y, qui se fixe ensuite après insertion du monomère à l'extrémité de la chaîne polymérique, laquelle constitue une espèce dormante qui peut continuer à croître dès lors qu'il subsiste des monomères dans le milieu de polymérisation.

Outre la présence d'un ou plusieurs monomères tels que définis ci-dessus, l'étape de polymérisation b) se déroule, classiquement, en présence d'un sel métallique (par exemple, un halogénure métallique, tel qu'un halogénure de cuivre, comme le chlorure de cuivre) et d'un ligand organique.

On précise que, par ligand organique, on entend un composé organique comprenant au moins un doublet libre apte à venir combler une lacune électronique d'un élément métallique (en l'occurrence, dans notre cas, une lacune électronique sur l'élément métallique du sel susmentionné) pour former un complexe métallique.

A titre d'exemple, un ligand organique approprié peut être un composé appartenant à la famille des composés pyridines, tels que la bipyridine.

L'étape de polymérisation b) peut être réalisée, en outre, dans un mélange eau/solvant organique (par exemple, un solvant alcoolique) sous flux d'un gaz inerte (tel qu'un flux d'argon) pendant une température et durée appropriées pour engendrer la polymérisation.

Après l'étape de polymérisation peut s'ensuivre une ou plusieurs étapes de lavage, notamment pour éliminer les éléments métalliques provenant du sel métallique utilisé pour réaliser la polymérisation (par exemple, en utilisant une solution complexante, telle qu'une solution d'éthylènediaminetriacétate (EDTA)) et les monomères n'ayant pas réagi.

Le procédé de l'invention peut comprendre, après l'étape de polymérisation, une étape d'hydrolyse destinée à protoner les groupes conducteurs de protons, lorsqu'ils se présentent sous forme d'un sel (soit, en d'autres termes, cette étape consiste à remplacer les cations du sel par des atomes d'hydrogène).

Les masses molaires moyennes des polymères obtenus à l'issue de l'étape b) peuvent aller de 1000 à 1 000 000 g/mol, de préférence de 2000 à 200 000 g/mol.

Un procédé spécifique conforme à l'invention est un procédé, dans lequel :
- les particules sont des particules de platine liées à un matériau carboné, du type noir de carbone ;
- le composé amorceur est un composé de formule (I) susmentionnée ;
- le monomère est un monomère de formule (III) susmentionnée.

L'invention a aussi pour objet des particules susceptibles d'être obtenues par le procédé de l'invention, à savoir des particules comprenant un matériau apte à catalyser la réduction de l'oxygène ou l'oxydation de l'hydrogène, lesdites particules étant fonctionnalisées par des polymères comprenant au moins un motif répétitif porteur d'au moins un groupe conducteur de protons, ces polymères étant liés auxdites particules *via* un groupe espaceur, qui est un reste de composé amorceur d'une polymérisation du type ATRP lié de façon covalente auxdites particules (le reste correspondant au reste de composé amorceur, après que celui-ci a réagi d'une part, au moyen d'un de ses groupes, avec les particules pour former une liaison covalente et d'autre part au moyen d'un autre de ses groupes avec un monomère) moyennant quoi les polymères se trouvent liés de façon covalente au reste de composé amorceur, et lesdites particules étant, en outre, liées, par exemple, de façon covalente, à un matériau carboné.

De telles particules sont particulièrement intéressantes, car elles permettent de transposer la phénoménologie du point triple à l'échelle moléculaire, le rôle du catalyseur étant rempli par le matériau constitutif de la particule en tant que telle, le rôle du conducteur protonique étant rempli par les polymères susmentionnés et le rôle du conducteur électronique étant rempli par le matériau carboné. Les liaisons covalentes entre le conducteur électronique et le catalyseur d'une part et entre le matériau conducteur protonique et le catalyseur d'autre part assurent, premièrement, un meilleur transfert des charges (respectivement, électrons et protons) et donc de meilleures performances et, deuxièmement, une parfaite stabilité en conditions de fonctionnement en pile, lorsque ces particules sont utilisées dans des piles. Ces deux résultats permettent de réduire le taux de chargement en catalyseur pour des performances accrues.

Comme déjà mentionné pour le procédé, les particules comprenant un matériau apte à catalyser la réduction de l'oxygène ou l'oxydation de l'hydrogène peuvent être des particules métalliques, à savoir des particules comprenant un ou plusieurs éléments métalliques (auquel cas, lorsqu'il y a plusieurs éléments métalliques, on pourra parler de particules en alliage(s) métallique(s).

Des particules métalliques particulièrement appropriées peuvent être des particules comprenant un métal noble, tel que le platine, le ruthénium, le palladium et des mélanges de ceux-ci.

Lorsque les particules obtenues selon l'invention sont destinées à être utilisées dans des piles PEMFC, les particules métalliques sont avantageusement des particules en platine.

Les restes de composé amorceur peuvent être des restes d'un composé amorceur tel que défini pour le procédé ci-dessus, en particulier, un composé comportant un groupe disulfure -S-S- formant pont entre deux parties de ce composé, au moins une de ces deux parties comportant un groupe phényle porteur d'un groupe amide -NH-CO-R¹, R¹ étant un groupe hydrocarboné porteur d'au moins un atome d'halogène (il s'entend, dans ce cas, que le groupe amide est lié à un atome de carbone du groupe phényle *via* l'atome d'azote du groupe -NH-).

Les polymères comprenant au moins un motif répétitif porteur d'au moins un groupe conducteur de protons sont similaires à ceux déjà décrits pour le procédé ci-dessus et peuvent être, en particulier, des polymères appartenant à la famille des polysulfones, polyéthercétones, polyphénylènes, polystyrènes, polymères aliphatiques fluorés, étant entendu que ces polymères doivent comporter des groupes conducteurs de protons, par exemple des groupes acide sulfonique, acide phosphonique ou acide carboxylique.

Quant au matériau carboné, il peut être du graphite, du noir de carbone, des fibres de carbone, des tubes de carbone (tels que des nanotubes de carbone), du graphène et les mélanges de ceux-ci.

A titre d'exemple, lorsque les particules sont des particules de platine, le composé amorceur est un composé de formule (I), le monomère est un monomère de formule (III) et le matériau carboné est du noir de carbone, les particules résultantes sont des particules de platine, auxquelles sont liés de façon covalente, des restes du composé amorceur de formule suivante : 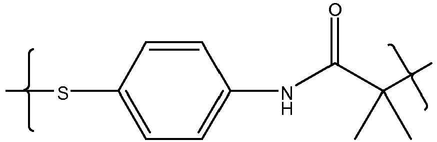 ce reste étant lié aux particules *via* l'atome de soufre, l'autre extrémité étant liée à des polymères comprenant un enchaînement de motifs répétitifs issus de la polymérisation du monomère de formule (III). Les masses molaires moyennes en masse des polymères greffés à la surface des particules peuvent aller de 1000 à 1 000 000 g/mol, idéalement de 2000 à 200 000 g/mol.

Le rapport entre matériau carbone et platine peut être compris entre 80/20 et 20/80, idéalement entre 45/55 et 65/35.

Les particules de l'invention peuvent entrer dans la constitution d'électrodes de piles à combustible, plus particulièrement, de couches catalytiques de piles à combustibles du type PEMFC, comme déjà mentionné ci-dessus.

En effet, ces particules sont très stables jusqu'à 200°C et présentent une tenue électrochimique jusqu'à 1 V v. ERH (ERH signifiant électrode réversible à dihydrogène).

Ainsi, l'invention a trait également à un dispositif de pile à combustible, par exemple du type PEMFC, comprenant au moins un assemblage électrode-membrane-électrode, dans lequel au moins une de ces électrodes est à base de particules conforme à l'invention.

La membrane, quant à elle, peut être à base d'un matériau polymérique conducteur de protons, le ou les polymères constitutifs de ce matériau pouvant être de même nature que le ou les polymères greffés à la surface desdites particules.

Parmi les composés amorceurs utilisés dans le cadre de cette invention, certains sont nouveaux, ces composés amorceurs étant des composés comportant un groupe disulfure -S-S- formant pont entre deux parties de ces composés, au moins une de ces deux parties comportant un groupe phényle porteur d'un groupe amide -NH-CO-R¹, R¹ étant un groupe hydrocarboné porteur d'au moins un atome d'halogène (il s'entend, dans ce cas, que le groupe amide est lié à un atome de carbone du groupe phényle *via* l'atome d'azote du groupe -NH-).

Les deux parties susmentionnées peuvent être identiques.

Un composé particulier répondant à la définition donnée ci-dessus peut être un composé de formule (I) ci-dessous :

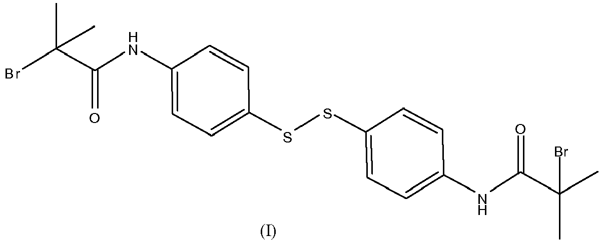

L'invention va être, à présent, décrite en référence aux exemples donnés ci-dessous à titre illustratif et non limitatif.

### EXPOSÉ DÉTAILLÉ DE MODES DE RÉALISATION PARTICULIERS

### EXEMPLE 1

Cet exemple illustre la préparation du composé 2-bromo-N-{4-[4-(2-bromo-2-méthylpropionylamino)-phényldisulfanyl]-phényl}-2-méthylpropionamide de formule (I) suivante : 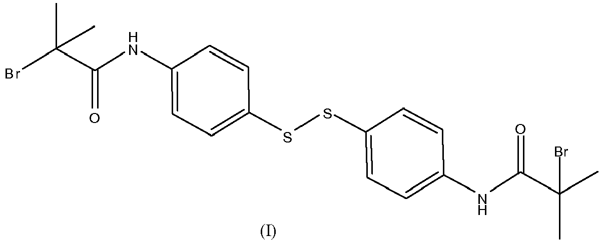 selon le schéma réactionnel suivant :

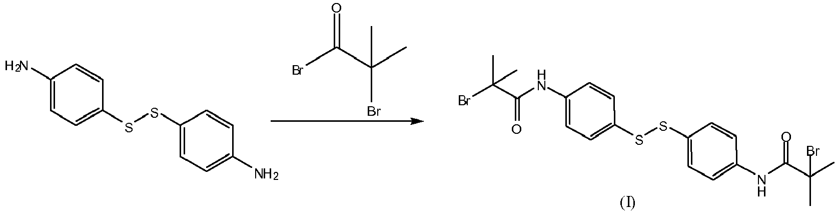

Pour ce faire, dans un ballon muni d'un barreau aimanté, du 4-aminophényle disulfure (248 mg ; 1 mmol ; 1 éq.) et de la bipyridine (343,6 mg ; 2,2 mmol ; 2,2 éq.) sont dissous dans du chloroforme (10 mL). Le mélange réactionnel est refroidi en plaçant le ballon dans un bain à 0°C. Du bromoisobutyrate (272 µL ; 2,2 éq.) est ajouté goutte-à-goutte au mélange ainsi refroidi. Le mélange résultant est agité pendant 10 heures puis on laisse la température remonter à la température ambiante. De l'eau (10 mL) est versée dans le ballon et le mélange résultant est ensuite transféré dans une ampoule à décanter, de sorte à séparer une phase aqueuse et une phase organique.

La phase aqueuse est lavée avec du dichlorométhane, moyennant quoi on isole une autre phase organique.

Les phases organiques sont réunies puis séchées avec du sulfate de magnésium (MgSO₄) puis filtrées. La phase résultante est ensuite évaporée sous vide à l'évaporateur rotatif, de sorte à éliminer les solvants organiques. Le produit résultant est purifié sur une colonne chromatographique sur gel de silice avec, dans un premier temps, du dichlorométhane comme éluant puis, dans un deuxième temps, un mélange dichlorométhane/méthanol comme éluant (90/10).

Le produit résultant (avec un rendement de 92%) correspond au produit attendu de formule (I) ci-dessus selon les analyses de spectroscopie RMN ¹H, de spectroscopie RMN ¹³C, de spectroscopie IR et d'analyse élémentaire, dont les résultats figurent ci-dessous.
**RMN ¹H** (200,13 MHz, CDCl₃) δ: 8,47 (s, 1H, NH), 7,56-7,42 (m, 4H, Hₐᵣₒₘ), 2,04 (s, 6H, CH₃) ppm.
**RMN ¹³C (200,13 MHz, CDCl₃) δ**: 170,1 (C=O), 137,2 (NH-*Cₐᵣₒₘ*), 132, 9 (S-*Cₐᵣₒₘ*), 130, 1 (HN-Cₐᵣₒₘ-CHₐᵣₒₘ-CHₐᵣₒₘ-S), 120,6 (H₂N-Cₐᵣₒₘ-*C*Hₐᵣₒₘ-CHₐᵣₒₘ-S), 63,1 (Br-*C*-(CH₃)₂, 32,6 (CH₃) ppm.
**IR (cm**⁻**¹)** : 3300 (N-H), 1615 (C=O), 1086 (C-S), 570 (C-Br).
**Analyse élémentaire (en %)** : (C₂₀H₂₂Br₂N₂O₂S₂), C: 42,5; H: 4,2; Br:30; N: 4,3; O: 7,9; S: 11,1.

### EXEMPLE 2

Cet exemple illustre la préparation de particules de platine liées à un matériau carboné, du type noir de carbone, (dénommé, dans la formule ci-dessous "Vulcan XC72"), réprésentées par la formule ci-dessous: 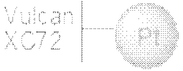 par une méthode impliquant une microémulstion dite "eau-dans-huile".

Cette préparation est menée, en parallèle, dans deux réacteurs séparés. Dans chaque réacteur, on verse de l'heptane (18,71 g ; 186,6 mmol) et du Brij® 30 (5,30 g ; 14,62 mmol). En parallèle, un sel de platine hexahydraté H₂PtCl₆.6H₂O (275 mg ; 0,212 mmol, 1 éq.) est dissous dans 2,5 mL d'eau milliQ. On ajoute, dans chaque réacteur, 1 mL de la solution de sel métallique puis on agite l'ensemble de sorte à former une microémulsion. Le mélange résultant est laissé au repos pendant une durée allant de 15 à 20 minutes. Du borohydrure de sodium (116 mg ; 3,1 mmol; 15 éq.) est ajouté par moitié dans chaque réacteur. Le mélange passe de l'orange au noir intense. Après agitation, on ajoute l'autre partie du borohydrure de sodium. Le mélange résultant est agité manuellement puis laissé au repos pendant 30 minutes. La réaction de réduction est considérée comme achevée, dès lors qu'il n'y a plus de dégagement gazeux. Le contenu des deux réacteurs est réuni dans un bécher, ce dernier étant recouvert de papier aluminium puis placé dans un bain à ultrasons pendant 10 minutes. Du noir de carbone Vulcan®XC 72 (120 mg) est ajouté dans le bécher. Ce dernier est replacé dans un bain à ultrasons pendant 30 minutes. Une fois le bêcher retiré du bain à ultrasons, de l'acétone est ajouté (1 volume d'acétone pour un volume de microémulsion). Le mélange est laissé au repos quelques minutes puis est filtré sur une membrane en polyfluorure de vinylidène (PVDF) hydrophile Durapore (0,22 µm; GVWP 04700) sous vide. Les particules de platine supportées sur le matériau carboné (le noir de carbone) sont lavées par filtration par cycles de 3*30 mL d'acétone, 3*30 mL d'un mélange acétone/eau (50/50), 3*30 mL d'eau dans un premier temps puis, dans un deuxième temps, par des séries (au moins trois) de 2*30 mL d'acétone, 1*30 mL d'un mélange acétone/eau (50/50) et 2*30 mL d'eau. Les particules obtenues sont alors placées une nuit à l'étuve à une température de 75°C.

Le rendement est quantitatif.

Les particules obtenues sont analysées par analyse élémentaire attestant de la présence de carbone (à hauteur de 60%) et de platine (à hauteur de 40%), ce qui démontre que les particules de platine sont supportées sur le matériau carboné.

### EXEMPLE 3

Cet exemple illustre la préparation de particules de platine liées à un matériau carboné du type noir de carbone (dénommé, dans la formule ci-dessous "Vulcan CX 72") par une méthode dite "méthode instantannée" (ou en terminologie anglaise "Instant method"), ces particules pouvant être représentées par la formule ci-dessous:

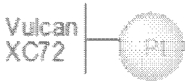

Pour ce faire, du sel de platine hexahydraté H₂PtCl₆.6H₂O (259 mg; 0,50 mmol; 1 éq.) est dissous dans 10 mL d'eau milliQ, moyennant quoi l'on obtient une solution de sel métallique. En parallèle, dans un ballon de 100 mL muni d'un barreau aimanté, du carbonate de lithium (111 mg; 1,5 mmol; 3 éq.) est dissous dans 30 mL d'eau milliQ. Du matériau carboné Vulcan®XC72 (146 mg) (correspondant à du noir de carbone) est introduit dans le ballon suivi de la solution de sel métallique. Pour obtenir une concentration finale en métal de 10 mmol.L⁻¹, 10 mL d'eau sont additionnés. Le pH du mélange est ajusté à une valeur de 9-10 par ajout de carbonate de lithium. Le mélange réactionnel est agité à 500 tours/min pendant 6 heures à 60°C. Après 6 heures de réaction, les particules d'oxyde de platine obtenues sont réduites par ajout d'une solution froide à 100 mmol.L⁻¹ de borohydrure de sodium (NaBH₄) (19 mg; 0,5 mmol; 1 éq.). L'étape de réduction est réalisée sous flux contrôlé à l'aide d'une pompe, dont le débit est fixé à 0,15 mL.min⁻¹. Une fois revenu à température ambiante, le mélange réactionnel est filtré sous vide puis lavé trois fois avec de l'eau milliQ. Des particules sont récupérées par filtration puis sont séchées une nuit à l'étuve à 75°C.

Le rendement est quantitatif.

Les particules obtenues sont analysées par analyse élémentaire attestant de la présence de carbone (à hauteur de 60%) et de platine (à hauteur de 40%), ce qui démontre que les particules de platine sont supportées sur le matériau carboné.

### EXEMPLE 4

Cet exemple illustre la préparation de particules de platine préparées selon les exemples précédents greffées par le composé préparé à l'exemple 1, ces particules greffées pouvant être schématisées par la formule ci-dessous:

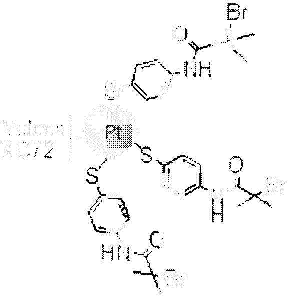

Pour ce faire, dans un ballon muni d'un barreau aimanté, les particules de platine sont mises en suspension dans 10 mL d'hexylamine. Le ballon est placé ensuite 30 minutes dans un bain à ultrasons, afin d'obtenir une dispersion desdites particules. Le composé de l'exemple 1 est dissous dans l'eau puis ajouté dans le ballon. Le mélange réactionnel est agité pendant 12 heures. Les particules de platine sont ensuite lavées, récupérées par des cycles de précipitation/centrifugation puis séchées une nuit à l'étude à 75°C.

Différents essais ont été conduits avec différentes quantités de particules et de composé de l'exemple 1, ces quantités étant illustrées dans le tableau 1 ci-dessous.

**Tableau 1**

| | | | | | | | | | | |
|---|---|---|---|---|---|---|---|---|---|---|
| Particules (mg) | 98 | 96 | 94 | 92 | 90 | 88 | 86 | 84 | 82 | 80 |
| Composé exemple 1 (mg) | 4 | 8 | 12 | 16 | 20 | 24 | 28 | 32 | 36 | 40 |

### EXEMPLE 5

Cet exemple illustre la préparation de particules de platine supportées sur un matériau carboné et greffées par un composé conforme à l'exemple 1, le greffage en tant que tel étant réalisé dans le milieu de synthèse des particules de platine supportées (ce qui permet de qualifier ce greffage de greffage *in situ*).

Les particules ainsi obtenues peuvent être schématisées par la même formule que celle représentée à l'exemple 4.

Pour ce faire, cette préparation est menée, en parallèle, dans deux réacteurs séparés. Dans chaque réacteur, on verse de l'heptane (18,71 g ; 186,6 mmol) et du Brij® 30 (5,30 g ; 14,62 mmol). En parallèle, un sel de platine hexahydraté H₂PtCl₆.6H₂O (275 mg ; 0,212 mmol, 1 éq) est dissous dans 2,5 mL d'eau milliQ. On ajoute, dans chaque réacteur, 1 mL de la solution de sel métallique puis on agite l'ensemble de sorte à former une microémulsion. Le mélange résultant est laissé au repos pendant une durée allant de 15 à 20 minutes. Du borohydrure de sodium (116 mg ; 3,1 mmol; 15 éq.) est ajouté par moitié dans chaque réacteur. Le mélange passe de l'orange au noir intense. Après agitation, on ajoute l'autre partie du borohydrure de sodium. Le mélange résultant est agité manuellement puis laissé au repos pendant 30 minutes. La réaction de réduction est considérée comme achevée, dès lors qu'il n'y a plus de dégagement gazeux. Le composé préparé à l'exemple 1 préalablement dissous est ajouté dans chaque réacteur. Les réacteurs sont placés sous agitation pendant 1 heure. Le contenu des deux réacteurs est ensuite réuni dans un bécher, ce dernier étant recouvert de papier aluminium puis placé dans un bain à ultrasons pendant 10 minutes. Du noir de carbone Vulcan®XC 72 (120 mg) est ajouté dans le bécher. Ce dernier est replacé dans un bain à ultrasons pendant 30 minutes. Une fois le bécher retiré du bain à ultrasons, de l'acétone est ajouté (1 volume d'acétone pour un volume de microémulsion). Le mélange est laissé au repos quelques minutes puis est filtré sur une membrane en polyfluorure de vinylidène (PVDF) hydrophile Durapore (0,22 µm; GVWP 04700) sous vide. Les particules de platine liées au matériau carboné (le noir de carbone) sont lavées par filtration par cycles de 3*30 mL d'acétone, 3*30 mL d'un mélange acétone/eau (50/50), 3*30 mL d'eau dans un premier temps puis, dans un deuxième temps, par des séries (au moins trois) de 2*30 mL d'acétone, 1*30 mL d'un mélange acétone/eau (50/50) et 2*30 mL d'eau. Les particules obtenues sont alors placées une nuit à l'étuve à une température de 75°C.

Différents essais ont été conduits avec différentes quantités de matériau carboné Vulcan XC®72 et de composé de l'exemple 1, ces quantités étant explicitées dans le tableau 2 ci-dessous.

**Tableau 2**

| | | | | | | | | | | |
|---|---|---|---|---|---|---|---|---|---|---|
| Vulcan®XC 72 (mg) | 58 | 56 | 54 | 52 | 50 | 48 | 46 | 44 | 42 | 40 |
| Composé exemple 1 (mg) | 4 | 8 | 12 | 16 | 20 | 24 | 28 | 32 | 36 | 40 |

### EXEMPLE 6

Cet exemple illustre la polymérisation du styrènesulfonate de sodium à partir des particules de platine préparées selon les exemples précédents, les particules ainsi obtenues pouvant être réprésentées par la formule ci-dessous: 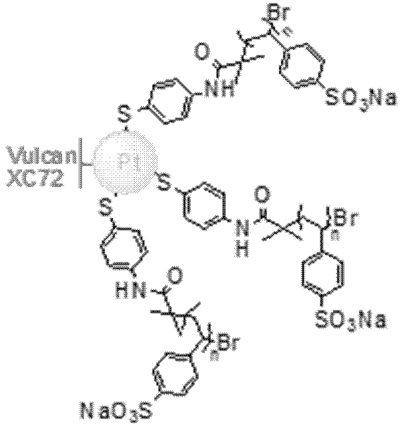 n correspondant au nomre de répétitition du motif pris entre parenthèses.

Pour ce faire, dans un bicol muni d'un barreau aimanté, les particules de platine greffées préparées préalablement (1 éq.) sont dispersées dans un mélange eau/méthanol (3/1) au moyen d'un bain à ultrasons (pendant 1 heure). Le mélange réactionnel est dégazé par application de cycles vide/argon. Du chlorure de cuivre (4 éq.) et de la 2,2'-bipyridine (8 éq.) sont ajoutés au mélange sous flux d'argon. Du styrènesulfonate de sodium est ajouté à la dispersion sous agitation et sous flux d'argon. Après 20 heures de polymérisation, la réaction est stoppée par mise à l'air du milieu réactionnel. Les particules de platine ainsi fonctionnalisées sont récupérées et lavées par plusieurs cycles de centrifugation en utilisant une solution d'EDTA (2*10 g.L⁻¹) et de l'eau milliQ (2 fois), afin d'éliminer les ions Cu²⁺ et les monomères restants. Le produit obtenu est séché à l'étuve pendant une nuit à 75°C.

Différents essais ont été mis en oeuvre de sorte à obtenir une masse molaire visée, les quantités de monomères nécessaires à l'obtention de la masse molaire visée étant indiquées dans le tableau 3 ci-dessous.

**Tableau 3**

| Masse molaire visée (g.mol-1) | Nombre d'équivalents de monomère |
|---|---|
| 10 000 | 48 |
| 50 000 | 240 |
| 100 000 | 480 |

Les particules obtenues ont été testées par analyse thermogravimétrique, de sorte à estimer la tenue thermique de celles-ci, cette analyse ayant été réalisée sous air avec une variation de températures allant de 25°C à 800°C.

Cette analyse a mis en évidence que, jusqu'à 200°C, aucune dégradation n'est visible ce qui permet d'envisager l'utilisation de ces particules pour entrer dans la constitution de couches catalytiques d'électrodes pour piles à combustible.

Il a également été procédé à la détermination des caractéristiques électrochimiques des particules obtenues ci-dessus.

En particulier, il a été procédé à la caractérisation de la tenue de la couronne organique en milieu support (la couronne organique correspondant aux polymères greffés aux particules *via* les restes de composé amorceur) avec une cellule à trois électrodes. Dans cette cellule, l'électrode de référence est une électrode réversible à dihydrogène (ERH) dont le potentiel électrochimique est fixé et connu. La seconde électrode est une électrode auxiliaire appelée contre électrode (CE) constituée d'un matériau inerte, une plaque de carbone vitreux dans notre cas, et qui sert à la collection du courant. La troisième électrode est une électrode de travail (ET) sur laquelle se trouve le catalyseur étudié. Un système d'entrée et de sortie de gaz (AG/SG) est ajouté afin de travailler en atmosphère contrôlée. La caractérisation de cette tenue a été effectuée par cyclage du potentiel d'électrode entre 0,05 V vs ERH (ERH signifiant électrode réversible à dihydrogène) et une limite supérieure de potentiel successivement égale à 0,55 V vs ERH (seuls les phénomènes faradiques d'adsorption/désorption d'hydrogène se produisant sur cette gamme de potentiels, les courants enregistrés entre 0,4 V et 0,55 V vs. ERH étant dus au phénomène de capacité de double couche), 0,8 V vs ERH (potentiel situé juste avant le début de la réaction d'oxydation du platine), 1,0 V vs ERH (potentiel situé après le début de la réaction d'oxydation du platine et correspondant au potentiel d'une cathode d'une pile du type PEMFC à circuit ouvert) et 1,2 V vs ERH (potentiel fortement oxydant).

Il ressort que, pour des potentiels d'électrode supérieures à 1,0 V vs ERH, les courants observés dans la zone d'adsorption/désorption augmentent jusqu'à l'obtention de valeurs de courant enregistrées sur une électrode à base de particules de platine uniquement liées à un matériau carboné (Vulcan XC72). Cette observation montre que la couronne organique, liée au platine est stable jusqu'à des potentiels appliqués de 1,0 V vs ERH.

Il a été procédé également à la caractérisation des particules de l'invention en milieu acide saturé en oxygène, de sorte à étudier leur comportement catalytique vis-à-vis de la réaction de réduction de l'oxygène. L'allure des voltammogrammes obtenus est équivalente à celle obtenue pour des catalyseurs constitués de particules de platine uniquement liées à un matériau carboné (Vulcan XC72). Le nombre total d'électrons échangés est égal à 4 entre 0,7 et 0,4 V vs ERH. La réduction de l'oxygène est donc complète pour former de l'eau.

Enfin, il a été procédé à la détermination de la sélectivité des particules de l'invention, cette sélectivité pouvant être définie comme la capacité d'un catalyseur à transformer des réactifs précis en un ou des produits donnés, qui sont, dans ce cas, pour la réduction de l'oxygène : l'eau et le peroxyde d'hydrogène. Il a été déterminé que le peroxyde d'hydrogène n'est produit qu'à partir de 0,8 V vs ERH. Cela implique une réduction directe de l'oxygène en eau dans la gamme de potentiels allant de 1,1 à 0,8 V vs ERH. Pour des potentiels inférieurs, la proportion de peroxyde s'élève jusqu'à 5%, ce qui est tout à fait compatible avec une utilisation en pile.

## Revendications

1. Procédé de préparation de particules comprenant un matériau apte à catalyser la réduction de l'oxygène ou l'oxydation de l'hydrogène, lesdites particules étant fonctionnalisées par des polymères comprenant au moins un motif répétitif porteur d'au moins un groupe conducteur de protons, ledit procédé comprenant successivement les étapes suivantes :
a) une étape de mise en contact de particules comprenant un matériau apte à catalyser la réduction de l'oxygène ou l'oxydation de l'hydrogène avec un composé amorceur d'une polymérisation du type ATRP, lequel composé comprend au moins un groupe apte à se greffer à la surface desdites particules, moyennant quoi l'on obtient des particules greffées par un reste dudit composé amorceur ;
b) une étape de mise en contact desdites particules obtenues en a) avec au moins un monomère porteur d'au moins un groupe conducteur de protons, moyennant quoi il y a polymérisation dudit monomère à partir des restes susmentionnés.

2. Procédé selon la revendication 1, dans lequel les particules sont des particules métalliques comprenant un métal noble, tel que le platine, le ruthénium, le palladium et des mélanges de ceux-ci.

3. Procédé selon l'une quelconque des revendications précédentes, dans lequel le composé amorceur est un composé organique halogénure comportant au moins un groupe choisi parmi -S-S- et -SH.

4. Procédé selon l'une quelconque des revendications précédentes, dans lequel le composé amorceur est un composé comportant un groupe disulfure -S-S- formant pont entre deux parties de ce composé, au moins une de ces deux parties comportant un groupe phényle porteur d'un groupe amide -NH-CO-R¹, R¹ étant un groupe hydrocarboné porteur d'au moins un atome d'halogène.

5. Procédé selon l'une quelconque des revendications précédentes, dans lequel le composé amorceur est un composé de formule (I) suivante :

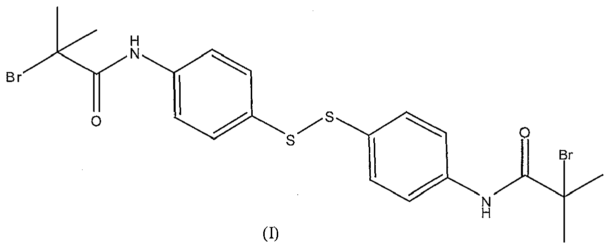

6. Procédé selon l'une quelconque des revendications précédentes, comprenant, avant l'étape a), une étape de préparation des particules comprenant un matériau apte à catalyser la réduction de l'oxygène ou l'oxydation de l'hydrogène.

7. Procédé selon la revendication, comprenant en outre, avant l'étape a), une étape de préparation de particules comprenant un matériau apte à catalyser la réduction de l'oxygène ou l'oxydation de l'hydrogène liées à un matériau carboné.

8. Procédé selon la revendication 1, comprenant, après l'étape a) et avant l'étape b), une étape de mise en contact des particules obtenues à l'issue de l'étape a) avec le matériau carboné, moyennant quoi lesdites particules sont liées, à l'issue de cette étape de mise en contact, au matériau carboné.

9. Procédé selon l'une quelconque des revendications précédentes, dans lequel le groupe conducteur de protons est un groupe acide sulfonique -SO₃H, un groupe acide carboxylique -CO₂H ou un groupe acide phosphonique -PO₃H₂, ces groupes pouvant être présents éventuellement sous forme de sels.

10. Procédé selon l'une quelconque des revendications précédentes, dans lequel le ou les monomères sont choisis de sorte à former, après polymérisation, des chaînes polymériques appartenant à la famille des polysulfones, polyéthercétones, polyphénylènes, polystyrènes, polyméres aliphatiques fluorés.

11. Procédé selon l'une quelconque des revendications précédentes, dans lequel le ou les monomères répondent à la formule (II) suivante : 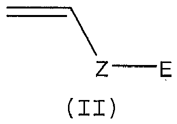 dans laquelle :
- Z correspond à un groupe phénylène ; et
- E correspond à un groupe conducteur de protons, éventuellement sous forme d'un sel, tel qu'un groupe acide sulfonique, un groupe acide phosphonique ou un groupe acide carboxylique.

12. Particules comprenant un matériau apte à catalyser la réduction de l'oxygène ou l'oxydation de l'hydrogène, lesdites particules étant fonctionnalisées par des polymères comprenant au moins un motif répétitif porteur d'au moins un groupe conducteur de protons, ces polymères étant liées auxdites particules *via* un groupe espaceur, qui est un reste de composé amorceur d'une polymérisation ATRP lié de façon covalente auxdites particules.

13. Particules selon la revendication 12, qui sont des particules métalliques comprenant un métal noble, tel que le platine, le ruthénium, le palladium et des mélanges de ceux-ci.

14. Particules selon la revendication 12 ou 13, pour lesquelles le reste est un reste d'un composé amorceur comportant un groupe disulfure -S-S- formant pont entre deux parties de ce composé, au moins une de ces deux parties comportant un groupe phényle porteur d'un groupe amide -NH-CO-R¹, R¹ étant un groupe hydrocarboné porteur d'au moins un atome d'halogène.

15. Particules selon l'une quelconque des revendications 12 à 14, pour lesquelles le ou les polymères appartiennent à la famille des polysulfones, polyéthercétones, polyphénylènes, polystyrènes, polymères aliphatiques fluorés, étant entendu que ces polymères doivent comporter des groupes conducteurs de protons.

## Patentansprüche

1. Verfahren zur Herstellung von Partikeln, umfassend ein Material, das dazu ausgelegt ist, die Reduktion von Sauerstoff oder die Oxidation von Wasserstoff zu katalysieren, wobei die Partikel durch Polymere funktionalisiert sind, die wenigstens ein sich wiederholendes Motiv umfassen, welches Träger wenigstens einer protonenleitenden Gruppe ist, wobei das Verfahren nacheinander die folgenden Schritte umfasst:
a) einen Schritt des Inkontaktbringens von Partikeln, umfassend ein Material, das dazu ausgelegt ist, die Reduktion von Sauerstoff oder die Oxidation von Wasserstoff zu katalysieren, mit einer Verbindung, die Initiator einer Polymerisation vom Typ ATRP ist, welche Verbindung wenigstens eine Gruppe umfasst, die dazu ausgelegt ist, sich an die Oberfläche der Partikel zu pfropfen, wodurch man Partikel erhält, die mit einem Rest der Initiatorverbindung gepfropft sind;
b) einen Schritt des Inkontaktbringens der in a) erhaltenen Partikel mit wenigstens einem Monomer, das Träger wenigstens einer protonenleitenden Gruppe ist, wodurch eine Polymerisation des Monomers ausgehend von den genannten Resten erfolgt.

2. Verfahren nach Anspruch 1, bei dem die Partikel Metallpartikel sind, umfassend ein Edelmetall wie z.B. Platin, Ruthenium, Palladium und deren Mischungen.

3. Verfahren nach einem der vorhergehenden Ansprüche, bei dem die Initiatorverbindung eine organische Halogenidverbindung ist, die wenigstens eine Gruppe ausgewählt aus -S-S- und -SH umfasst.

4. Verfahren nach einem der vorhergehenden Ansprüche, bei dem die Initiatorverbindung eine Verbindung ist, die eine Disulfidgruppe -S-S- umfasst, die eine Brücke bildet zwischen zwei Teilen dieser Verbindung, wobei wenigstens einer dieser zwei Teile eine Phenylgruppe umfasst, die Träger einer Amidgruppe -NH-CO-R¹ ist, wobei R¹ eine Kohlenwasserstoffgruppe ist, die Träger wenigstens eines Halogenatoms ist.

5. Verfahren nach einem der vorhergehenden Ansprüche, bei dem die Initiatorverbindung eine Verbindung mit nachfolgender Formel (I) ist:

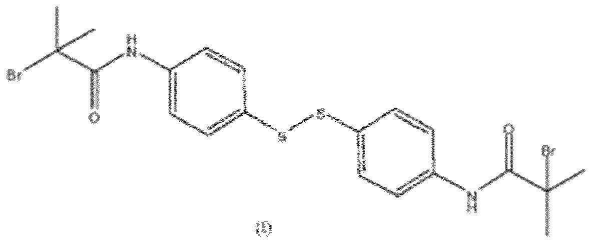

6. Verfahren nach einem der vorhergehenden Ansprüche, umfassend vor dem Schritt a) einen Schritt der Präparation der Partikel, umfassend ein Material, das dazu ausgelegt ist, die Reduktion von Sauerstoff oder die Oxidation von Wasserstoff zu katalysieren.

7. Verfahren nach Anspruch, ferner umfassend, vor dem Schritt a) einen Schritt der Präparation von Partikeln, umfassend ein Material, das dazu ausgelegt ist, die Reduktion von Sauerstoff oder die Oxidation von Wasserstoff zu katalysieren, die an ein Kohlenstoffmaterial gebunden sind.

8. Verfahren nach Anspruch 1, umfassend nach dem Schritt a) und vor dem Schritt b) einen Schritt des Inkontaktbringens der am Ende des Schritts a) erhaltenen Partikel mit dem Kohlenstoffmaterial, wodurch die Partikel am Ende dieses Schritts des Inkontaktbringens an das Kohlenstoffmaterial gebunden werden.

9. Verfahren nach einem der vorhergehenden Ansprüche, bei dem die protonenleitende Gruppe eine Sulfonsäuregruppe -SO₃H, eine Carboxylsäuregruppe -CO₂H oder eine Phosphonsäuregruppe -PO₃H₂ ist, wobei diese Gruppen gegebenenfalls in Form von Salzen vorhanden sein können.

10. Verfahren nach einem der vorhergehenden Ansprüche, bei dem das oder die Monomer(e) derart ausgewählt ist/sind, dass nach der Polymerisation Polymerketten gebildet werden, die zur Familie der Polysulfone, der Polyetherketone, der Polyphenylene, der Polystyrole, der aliphatischen Fluorpolymere gehören.

11. Verfahren nach einem der vorhergehenden Ansprüche, bei dem das oder die Monomer(e) der nachfolgenden Formel (II) genügt/genügen: 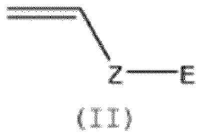 wobei:
- Z einer Phenylengruppe entspricht; und
- E einer protonenleitenden Gruppe entspricht, gegebenenfalls in Form eines Salzes, wie z.B. eine Sulfonsäuregruppe, eine Phosphonsäuregruppe oder eine Carboxylsäuregruppe.

12. Partikel, umfassend ein Material, das dazu ausgelegt ist, die Reduktion von Sauerstoff oder die Oxidation von Wasserstoff zu katalysieren, wobei die Partikel durch Polymere funktionalisiert sind, die wenigstens ein sich wiederholendes Motiv umfassen, das Träger wenigstens einer protonenleitenden Gruppe ist, wobei diese Polymere an die Partikel über eine Spacergruppe gebunden sind, die ein Rest der Initiatorverbindung einer ATRP-Polymerisation ist, die kovalent an die Partikel gebunden ist.

13. Partikel nach Anspruch 12, die Metallpartikel sind, umfassend ein Edelmetall, wie z.B. Platin, Ruthenium, Palladium und deren Mischungen.

14. Partikel nach Anspruch 12 oder 13, bei denen der Rest ein Rest einer Initiatorverbindung ist, umfassend eine Disulfidgruppe -S-S-, die eine Brücke zwischen zwei Teilen dieser Verbindung bildet, wobei wenigstens einer dieser zwei Teile eine Phenylgruppe umfasst, die Träger einer Amidgruppe -NH-CO-R¹ ist, wobei R¹ eine Kohlenwasserstoffgruppe ist, die Träger wenigstens eines Halogenatoms ist.

15. Partikel nach einem der Ansprüche 12 bis 14, für die das oder die Polymer(e) zur Familie der Polysulfone, der Polyetherketone, der Polyphenylene, der Polystyrole, der aliphatischen Fluorpolymere gehört/gehören, wobei diese Polymere protonenleitende Gruppen enthalten müssen.

## Claims

1. Method for preparing particles comprising a material capable of catalyzing the reduction of oxygen or the oxidation of hydrogen, said particles being functionalized by polymers comprising at least one repeating unit bearing at least one proton-conducting group and said particles being covalently bonded to a carbon material, said method comprising the following consecutive steps:
a) a step of contacting particles, comprising a material capable of catalyzing the reduction of oxygen or the oxidation of hydrogen, with a compound that is an initiator for ARTP polymerization, which compound comprises at least one group capable of being grafted onto the surface of said particles, whereby particles, onto which a radical of said initiator compound is grafted, are obtained;
b) a step of contacting said particles obtained in a) with at least one monomer bearing at least one proton-conducting group, whereby there is polymerization of said monomer from the above-mentioned radicals.

2. Method according to claim 1, in which the metal particles are particles comprising a noble metal, such as platinum, ruthenium, palladium and mixtures thereof.

3. Method according to any of the preceding claims, in which the initiator compound is an organic halide compound comprising at least one group selected from -S-S- and -SH.

4. Method according to any of the preceding claims, in which the initiator compound is a compound comprising a disulfide group -S-S- forming a bridge between two portions of said compound, at least one of said two portions comprising a phenyl group bearing an amide group -NH-CO-R¹, R¹ being a hydrocarbon group having at least one halogen atom.

5. Method according to any of the preceding claims, in which the initiator compound is a compound of following formula (I):

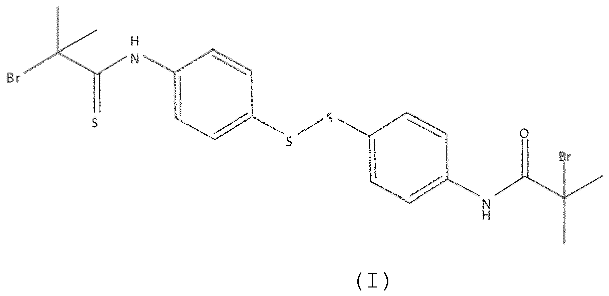

6. Method according to any of the preceding claims, comprising, before step a), a step of preparing particles comprising a material capable of catalyzing the reduction of oxygen or the oxidation of hydrogen.

7. Method according to claim 1 or 11, further comprising, before step a), a step of preparing particles comprising a material capable of catalyzing the reduction of oxygen or the oxidation of hydrogen bound to a carbon material.

8. Method according to claim 1, comprising, after step a) and before step b), a step of contacting the particles obtained at the end of step a) with the carbon material, whereby said particles are bound, at the end of said contacting step, to the carbon material.

9. Method according to any of the preceding claims, in which the proton-conducting group is a sulfonic acid group -SO₃H, a carboxylic acid group -CO₂H or a phosphonic acid group-PO₃H₂, said groups being able to be present potentially in the form of salts.

10. Method according to any of the preceding claims, in which the monomer (s) are chosen so as to form, after polymerization, polymer chains belonging to the family of polysulfones, polyetherketones, polyphenylenes, polystyrenes, fluorinated aliphatic polymers.

11. Method according to any of the preceding claims, in which the monomer(s) meet the following formula (II): 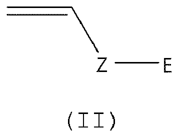 in which:
- Z corresponds to a phenyl group; and
- E corresponds to a proton-conducting group, potentially in the form of a salt, such as a sulfonic acid group, a phosphonic acid group or a carboxylic acid group.

12. Particles comprising a material capable of catalyzing the reduction of oxygen or the oxidation of hydrogen, said particles being functionalized by polymers comprising at least one repeating unit having at least one proton-conducting group, said polymers being bound to said particles via a spacer group, which is a radical of a compound that is an initiator for ATRP polymerization bound in a covalent manner to said particles and said particles being bound in a covalent manner to a carbon material.

13. Particles according to claim 12, which are particles comprising a noble metal, such as platinum, ruthenium, palladium and mixtures thereof.

14. Particles according to any of claims 12 to 13, for which the radical is a radical of an initiator compound comprising a disulfide group -S-S- forming a bridge between two portions of said compound, at least one of said two portions comprising a phenyl group bearing an amide group -NH-CO-R¹, R¹ being a hydrocarbon group bearing at least one halogen atom.

15. Particles according to any of claims 12 to 14, for which the polymer(s) belong to the family of polysulfones, polyetherketones, polyphenylenes, polystyrenes, fluorinated aliphatic polymers, it being understood that said polymers comprise proton-conducting groups.
